Europäisches Patentamt

European Patent Office

Office européen des brevets

(11) Publication number: 0 349 348
A1

# EUROPEAN PATENT APPLICATION

(21) Application number: 89306697.7

(22) Date of filing: 30.06.89

(51) Int. Cl.⁵: **C 02 F 3/12**
C 02 F 3/34

(30) Priority: 01.07.88 DK 3647/88

(43) Date of publication of application:
03.01.90 Bulletin 90/01

(84) Designated Contracting States:
AT BE CH DE ES FR GB GR IT LI LU NL SE

(71) Applicant: NOVO-NORDISK A/S
Novo Allé
DK-2880 Bagsvaerd (DK)

(72) Inventor: Invorsen, Kjeld
Klostergaardsvej 35
DK-3500 Vaerloese (DK)

(74) Representative: Brown, John David et al
FORRESTER & BOEHMERT Widenmayerstrasse 4/I
D-8000 München 22 (DE)

The applicant has filed a statement in accordance with Rule 28 (4) EPC (issue of a sample only to an expert). Accession number(s) of the deposit(s): DSM 4009 + DSM 4010.

(54) Process for decomposition of metal-cyano complexes using microbial enzymes.

(57) An enzymatic process for specific decomposition of metal-cyano complexes in waste waters and process streams wherein microbial enzymes which are not oxygenases and which do not require the regeneration of coenzymes or prostetic groups are brought into contact with said waste water or process stream for a period sufficient to decompose said metal-cyano complexes to a desired concentration.

# Description

## Process for decomposition of metal-cyano complexes using microbial enzymes.

### TECHNICAL FIELD OF THE INVENTION

The present invention relates to a process for the specific decomposition of metal-cyano complexes in industrial process streams and effluents using enzymes produced by microorganisms by which process the solution containing the metal-cyano complexes is contacted with metal-cyano complex degrading enzyme preparations obtained from microorgamisms.

### BACKGROUND OF THE INVENTION

Waste waters containing free cyanides (hereby defined as $CN^-$ and HCN) and cyanide-complexed metals are produced in enormous quantities by metal processing industries (e.g. case hardening of steel, electroplating and mining). Within these waste streams transition metals (e.g. Ni, Zn, Cu, Co, Fe, Cd, Au, and Ag) are mainly present as metal-cyano complexes of the general formula:
$$M_x(CN)_y^{z-}$$
wherein M is a metal and x, y, and z are integers greater than 0.

Metal-cyano complexes are in themselves, and also due to their metal and cyanide components highly toxic to the environment, and waste waters from metal processing factories constitute a serious pollution problem. Regulations usually require that the concentration of metal-cyano complexes in waste streams be reduced to around at least a few ppm (measured as metal) before discarge into natural ecosystems.

Normally treatment of these effluents involves a physico/chemical decomposition of the metal-cyano complexes and subsequent recovery of the metal e.g. by precipitation , electrolysis or ion exchange.

Treatment of effluents containing metal-cyano complexes has proven very difficult because of the high chemical stability of these complexes (dissociation constants ($K_c$) range from $10^{-21}$ to $10^{-64}$, cf. Dahl, F. 1979. Analysemetoder til bestemmelse af cyanidforbindelser i vand. Teknologisk Institut, Kemiteknik). Most metal-cyano complexes are not easily decomposed by conventional alcaline chlorination processes used for oxidation of free cyanide, and consequently very harsh and often expensive treatment methods are needed for efficient decomposition of metal-cyanide complexes with high stability constants (e.g. complexes with Ni, Co, Fe, Au and Ag).

Much research has been devoted to this particular area of waste treatment but to date no cheap and universal physico/chemical methods are available for efficient degradation of metal-cyano complexes in effluents.

It is known that sewage systems and acclimatized biofilters are able to treat effluents containing metal-cyano complexes. The efficiency of these bio-systems which usually require very long periods of acclimatization is highly variable and inconsistent. Furthermore, biological treatment processes being dependent on the action of live microorganisms only seem capable of handling effluents which contain low levels of metal-cyano complexes (i.e. generally less than 50-100 ppm metal cyanide complex, cf. Knowles C. J., Bact. Reviews, 40: 652-680, 1976).

Although capable of treating effluents containing metal-cyano complexes these systems are diffucult to optimize and maintain because the exact biochemical mechanisms of metal cyanide removal are not known. These systems are, therefore, in essence black boxes in which metal-cyano complexes are absorbed to organic matter, chemically converted, retained and/or biologically transformed. A further drawback of these systems is that transition metals are not easily recovered from the resulting sludge.

It has been proposed that single microbial enzymes may be applied for decomposition of metal-cyano complexes as a means of effluent detoxification and as a first step in the recovery of the metal ions.

A new enzyme - cyanide oxygenase - was recently described as obtained from Pseudomonas fluorescens NCIB 11764 by Rollinson and coworkers (FEMS Microbiology Letters 40: 199-205, 1987). Cyanide oxygenase catalyzes the reaction:
$$HCN + O_2 + NADH \rightarrow NH_3 + CO_2 + NAD^+$$
and thus has a requirement for a nicotinamide-adenine dinucleotide coenzyme.

Subsequent studies have shown that this enzyme was also able to decompose $Ni(CN)_4^{2-}$ and $Cu(CN)_4^{3-}$ (but not $Zn(CN)_4^{2-}$ and $Fe(CN)_6^{2-}$). The activity of cyanide oxygenase towards other metal-cyano complexes was not reported in the above mentioned research paper. Cyanide oxygenase is the first enzyme with demonstrated ability to decompose metal-cyano complexes. However due to its apparently limited substrate specificity and the requirement for continuous regeneration of reduced coenzyme its utility as a commercial enzyme for large scale detoxification seems rather limited.

The enzyme cyanide hydratase (enz. reaction: $HCN + H_2O \rightarrow HCONH_2$) is produced by many microorganisms (especially fungi) and patents have been applied for one such enzyme and its uses for removal of cyanide from various sources ( EP Publication Nos. 61 249, 116 423, 233 719, and 234 760). The above mentioned cyanide hydratase has been commercialized under the name Cyclear. There has never been any reports that cyanide hydratases posses any activity towards metal-cyanide complexes, and thus the above mentioned cyanide oxygenase is the only documented microbial enzyme which is able to decompose metal-cyano complexes.

### BRIEF DESCRIPTION OF THE INVENTION

The object of the present invention is consequently to provide for a simple process for the specific decomposition of metal-cyano complexes using microbial enzymes which are not oxygenases and which do not require the regeneration of coenzymes or prostetic groups.

It has now surprisingly been found that certain enzymes produced by microorganisms belonging to the genus Alcaligenes are able to decompose metal-cyano complexes when they are brought into contact with a waste water or process stream for a suitable period thereby bringing the concentration of said metal-cyano complexes to a desired level.

Specific examples of microorganisms that produce such enzymes are Alcaligenes sp. DSM 4010 and DSM 4009.

Alcaligenes sp. DSM 4009 and DSM 4010 are described and deposited in accordance with the Budapest Treaty in connection with Danish patent application No 1283/87 filed March 12, 1987, which is hereby incorporated by reference.

## DETAILED DESCRIPTION OF THE INVENTION

As indicated above the present invention relates to the specific decomposition of metal-cyano complexes (preferably those of transition metals) by microbial enzymes which do not have a requirement for a coenzyme.

The enzymes embodied by the present invention do not have the drawback of the known oxidoreductases which are dependent on a redox coenzyme which must be supplied continuously or regenerated during the enzymatic reaction a property which greatly restricts their technical use.

For simplicity metal-cyano complex degrading enzymes which do not require coenzymes (e.g. hydrogen or electron transferring coenzymes) are hereinafter abbreviated : M-CC degrading enzymes.

According to the present invention enzyme preparations which consist of live or dead microbial cells can be used for treating solutions containing high and toxic levels of metal-cyano complexes.

Furthermore, the process of the present invention is able to decompose both free cyanide and metal-cyano comple xes using enzyme systems derived from one single microorganism.

According to the present invention high concentrations of cyanide complexes of e.g. Ni, Cu, Co, Zn, Cd, Au, Ag, Fe, and Pd may be completely decomposed to yield the free metal ion which may in turn occur freely in solution or be absorbed by the enzyme preparation. The cyanide moiety of the metal-cyano complex may e.g. by converted to $CO_2$, $HCOOH$, $HCONH_2$ or other cellular metabolites. Ammonia may also be a product of the reactions.

Enzymes which can be used according to the present invention comprise for example M-CC degrading enzymes derived from Alcaligenes sp DSM 4010 and DSM 4009 (described and deposited in connection with Danish Patent Application No. 1283/87 filed March 12, 1987, which is hereby incorporated by reference. However, any microbially derived M-CC degrading enzymes (excluding cyanide oxygenases) having the desired properties may be applied to effect the present invention. The above mentioned microorganisms, therefore, only represent suggestions as to suitable microorganisms.

Thus any microbial M-CC degrading enzymes having the desired and claimed properties may be applied to effect the present invention.

For application of the present invention the active enzymes may be applied as crude fermentation broth or whole cells both of which may be freeze-dried. Also cell-free extracts or purified enzyme forms can be used. In the application of the enzymes, use can also be made of known immobilization and cross-linking techniques applied on whole cells or enzyme preparations.

Depending on the specific microbial strain used, the process is effected at temperatures within the range of from about 0°C to 55°C preferably 10°C, to 45°C with 37°C representing the optimum temperature under most circumstances. The preferred pH range of the enzymatic reaction is 5 to 11 and preferably between 6 and 8.5.

Concentrations of metal-cyano complexes which can be decomposed by the M-CC degrading enzymes range from less than 1 mM to 50 mM or more depending on the type of complexed ion. Such concentrations are typically reduced to a level below 1 μM or 0.1 μM or less.

By the process of the invention it is possible to work in effluents or process streams that contain up to at least 0.4 mM, even up to 4 mM, and most surprising up to 40 mM or more metal-cyano complexes.

In the following examples enzyme activities are expressed in International Units (hereinafter abbreviated IU). One IU of enzyme corresponds to the amount of enzyme which catalyzes the conversion of 1 μmole of free cyanide per minute in 60 mM NaCN at a pH value of 7.5 and a temperature of 25°C.

Concentrations of metal-cyano complexes were determined either by measuring UV absorbance (Rollinson, G. et al., 1987, FEMS Microbiol. Lett. 40, 199-205) or using established HPLC procedures (Hilton, D.F. & Haddad, P.R., 1986, J. Chromatogr. 361, 141-150).

All the examples mentioned below were also performed without addition of enzyme preparation. In all these controls no conversion of neither complexed nor free cyanide was observed.

Also, in the examples the detection limits for some of the various transition metal complexes were:

| | | |
|---|---|---|
| -Ag(CN)$_2^-$ : | 0.30 | μM |
| -Au(CN)$_2^-$ : | 0.14 | μM |
| -Ni(CN)$_4^{2-}$: | 1.00 | μM |
| -Cu(CN)$_4^{3-}$: | 0.3 | μM |

## Example 1.

Decomposition of Nickel cyanide

Washed whole cells or cross-linked cells of DSM 4009 in an amount of 4,500 IU/l was added to a solution obtained by dissolving 3 mMole $NiCl_2$ and 100 mMole NaCN per l water (probably present as 3 mM Ni(CN)$_4^{2-}$ (approximately 500 ppm) + 100 mM Na$^+$ + 88mM CN$^-$ + 6mMCl$^-$) in 0.1 M phosphate buffer at pH 7.8. The reaction mixture was incubated at room temperature (22°C) with gentle stirring.

After 48 hours the concentration of nickel cyanide was reduced to less than about 1 μM as determined by HPLC analysis, and both complexed and free cyanide had been converted to ammonia as evidenced by analysis for ammonia according to the method of Chaney and Marbach (Clinical Chemistry, 8: 130-132, 1962).

## Example 2

Decomposition of Nickel Cyanide

The test described in Example 1 was repeated with the exception that the reaction mixture was 9.5 mM $Ni(CN)_4^{2-}$ (appr. 1,600 ppm) (estimated as in Example 1) and 20 mM NaCN in 0.1 M phosphate buffer.

Using enzyme preparations prepared from DSM 4009, the concentration of nickel cyanide was less than about 0.4 mM after 48 hours and less than about 4 μM after 168 hours.

## Example 3

Decomposition of Nickel Cyanide

The test described in Example 1 was repeated with the exception that the reaction mixture was 20 mM $Ni(CN)_4^{2-}$ (appr. 3,200 ppm), 40 mM NaCN and contained 6,000 IU/l of enzyme.

Using enzyme preparations prepared from DSM 4009, the concentration of nickel cyanide was less than about 60 μM after 72 hours and less than about 10 μM after 128 hours.

## Example 4

Decomposition of Copper Cyanide

The test described in Example 1 was repeated with the exception that the reaction mixture was 9.3 mM $Cu(CN)_4^{3-}$ (appr. 1,600 ppm) (obtained by dissolving 10 mMole CuCN in 30 mMole NaCN per l water).

Using enzyme preparations prepared from DSM 4009, the concentration of copper cyanide was less than about o.3 μM after 26 hours.

## Example 5

Decomposition of Copper Cyanide

The test described in Example 1 was repeated with the exception that the reaction mixture was 26 mM $Cu(CN)_4^{3-}$ (appr. 4,400 ppm).

Using enzyme preparations prepared from DSM 4009, the concentration of copper cyanide was less than about 5 μM after 96 hours.

## Example 6

Decomposition of Zinc Cyanide

The test described in Example 1 was repeated with the exception that the reaction mixture was 4 mM $Zn(CN)_4^{2-}$ (appr. 700 ppm) and 10 mM NaCN.

Using enzyme preparations prepared from DSM 4009, zinc cyanide was completely decomposed (i.e. residual concentration less than about 5 μM) after 48 hours as evidenced by analysis of ammonia.

## Example 7

Decomposition of Zinc Cyanide

The test described in Example 6 was repeated with the exception that the reaction mixture contained 10 mM $Zn(CN)_4^{2-}$ (appr. 1,700 ppm) and 10 mM NaCN.

Using enzyme preparations prepared from DSM 4009, zinc cyanide was completely decomposed (i.e. residual concentration less than about 5 μM) after 62 hours as evidenced by analysis of ammonia.

## Example 8

Decomposition of Cyano Complexes of Transition Metals

The decomposition experiments of Examples 1 through 7 were repeated with the exception that the tests were conducted under strict anoxic conditions in closed reaction vessels pressurized with nitrogen. Decomposition rates obtained in these tests under oxygen exclusion were almost similar to those given in Examples 1 through 7.

## Example 9

Decomposition of Gold Cyanide

The test described in Example 1 was repeated with the exception that the reaction mixture was 1.5 mM AuCN (appr. 450 ppm), 10 mM NaCN and contained 6,000 IU/l of enzyme.

Using enzyme preparations prepared from DSM 4009, the concentration of gold cyanide was less than about 0.1 μM after 21 hours.

## Example 10

Decomposition of Silver Cyanide

The test described in Example 1 was repeated with the exception that the reaction mixture was 0.5 mM AgCN (appr. 100 ppm), 10 mM NaCN and contained 6,000 IU/l of enzyme.

Using enzyme preparations prepared from DSM 4009, the concentration of gold cyanide was less than about 0.3 μM after 21 hours.

## Example 11

Cultivation of strain DSM 4009

The strain was maintained on slants of nutrient broth containing 2% agar. Nutrient broth was purchased from Difco Laboratories (Detroit, Michigan, USA). Liquid culturing was carried out in 500 ml shake flasks containing 100 ml of nutrient broth medium supplemented with 1% glycerol. Incubation was at 30° C for approximately 24 hours with good aeration. Induction of cyanide converting enzymes was done after 8 - 10 hours of growth by adding NaCN (1 mM) to the growing culture. Bacterial cells were recovered by centrifugation, washed and stored at 4° C or freeze-dried. If desired, the cyanide converting enzymes may be isolated and purified in manners known per se.

Constitutive strains are cultivated as above, the only difference being that no induction of the cyanide converting enzyme production is necessary.

## Example 12

Production of Enzyme Preparation (Whole Cell) from DSM 4009

Cells cultivated as described in Example 11 were harvested by centrifugation and washed one to three times with 0.1 M phosphate buffer (pH 7).

Washed cell suspensions of different cell densities (and thus different activity) were stored at 4°C in 0.1 M phosphate buffer. Alternatively, washed cell suspensions were freeze-dried or stored at -25°C.

## Example 13

Production of an Immobilized Cell Preparation of DSM 4009

The cell bound cyanidase of DSM 4009 was immobilized by cross-linking whole washed cells (obtained as described in Example 11) using standard immobilization techniques using e.g. glutaraldehyde as cross-linking agent.

DSM 4009 and DSM 4010 were deposited at Deutsche Sammlung von Mikroorganismen, Göttingen, Federal Republic of Germany on 23 February 1987, for patent purposes under the conditions of the Budapest Treaty.

The features disclosed in the foregoing description, in the following claims and/or in the accompanying drawings may, both separately and in any combination thereof, be material for realising the invention in diverse forms thereof.

## Claims

1. A metal-cyano complex degrading enzyme complex of bacterial origin, not requiring the regeneration of a coenzyme or prostetic group, capable of being active in a metal-cyano complex concentration equal to or above 0.4 mM.

2. An enzyme complex according to Claim 1 capable of being active in a metal-cyano complex concentration equal to or above 1 mM.

3. An enzyme complex according to Claim 1 or 2 capable of being active in a metal-cyano complex concentration equal to or above 4 mM.

4. An enzyme complex according to Claim 1, 2 or 3 capable of being active in a metal-cyano complex concentration equal to or above 40 mM.

5. An enzyme complex according to any one of Claims 1 to 4 capable of depleting the content of metal-cyano complex to below 1 μM.

6. An enzyme complex according to any one of Claims 1 to 4 capable of depleting the content of metal-cyano complex to 0.1 μM or below.

7. An enzyme complex according to any one of Claims 1 to 6 capable of degrading metal-cyano complexes at temperatures from 0°C to 55°C, preferably 10°C to 45°C.

8. An enzyme complex according to any one of Claims 1 to 7 capable of degrading metal-cyano complexes at pH values from 5 to 11, preferably from 6 to 8.5.

9. A metal-cyano complex degrading enzyme complex, which is immunologically identical or partially identical immunologically with a metal-cyano complex degrading enzyme producable by Alcaligenes sp. accorded the accession No. DSM 4009, mutants or genetically engineererd derivatives hereof.

10. A metal-cyano complex degrading enzyme complex which is immunologically identical or partially identical immunologically with a metal-cyano complex degrading enzyme producable by Alcaligenes sp. accorded the accession No. DSM 4010, mutants or genetically engineered derivatives thereof.

11. An enzymatic process for decomposition of a metal-cyano complex in solution, wherein a metal-cyano complex degrading enzyme complex not requiring the regeneration of a coenzyme or prostetic group is brought into contact with the solution for a period sufficient to decompose the metal-cyano complex to a desired concentration.

12. Process according to Claim 11, wherein the metal-cyano complex belongs to the group comprising, as the metal ion at least one of the transition metals.

13. A process according to Claim 12, wherein the metal-cyano complex belongs to the group comprising as the metal ion at least one of: Ni, Cu, Au, Ag, Fe, Cd, Co, and Pd in any of their oxidation states.

14. A process according to Claims 12 or 13, wherein the microbial enzymes are derivable from one or more strains belonging to the genus Alcaligenes sp..

15. A process according to Claim 14, wherein said strains are accorded the accession No. DSM 4009 or DSM 4010, muants or genetically engineered derivatives thereof.

16. A process according to any one of Claims 11 to 15, wherein the enzymatic decomposition reaction takes place in solutions of pH in the range of from pH 5 to pH 11.

17. A process according to Claim 16, wherein the enzymatic decomposition reaction is capable of taking place in solutions of pH in the range of from pH 6 to pH 8.5.

18. A process according to any one of Claims 11 to 17 in which the enzymatic decomposition reaction is carried out at a temperature in the range of from 0°C to 55°C.

19. A process according to Claim 18 wherein the enzymatic decomposition reaction may be carried out at a temperature in the range of from 10°C to 45°C.

20. A process according to any one of Claims 11 to 19, wherein the metal-cyano complex is in solution in waste water and/or a process stream.

21. A metal-cyano degrading enzyme preparation, wherein the enzyme is present as intact cells, treated cells, a crude cell extract, or a pure enzyme either alone or in admixture with any suitable additive.

22. A preparation according to Claim 21, in cross-linked or immobilized form.

23. A preparation according to Claim 21 or 22, wherein the enzyme is applicable as chemically modified enzyme or chemically modified cells.

24. A preparation according to Claim 21, 22, or 23, wherein the enzyme is producable by a microbial strain belonging to the genus Alcaligenes sp., mutants or genetically engineered derivatives thereof.

25. A preparation according to Claim 24, wherein the strain is DMS 4009 or DSM 4010.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P<br>D | EP-A-0 282 351 (NOVO INDUSTRI)<br>* Claims 1,4,5,12,14,15,18-32; page 10, example 20; page 4, lines 13-40 * | 21-25 | C 02 F 3/12<br>C 02 F 3/34 |
| A,P<br>D | | 1-20 | |
| | --- | | |
| X,D | FEMS MICROBIOLOGY LETTERS, vol. 40, 1987, pages 199-205, Federation of European Microbiological Societies, Amsterdam, NL; G. ROLLINSON et al.: "The growth of a cyanide-utilising strain of Pseudomonas fluorescens in liquid culture on nickel cyanide as a source of nitrogen"<br>* Page 204, right-hand column, last paragraph - page 205 * | 21 | |
| | --- | | |
| A | US-A-4 440 644 (MUDDER et al.)<br>* Column 2: "Summary of the invention" * | 1 | |
| | ----- | | TECHNICAL FIELDS SEARCHED (Int. Cl.5)<br><br>C 02 F<br>C 22 B |

The present search report has been drawn up for all claims

| Place of search | Date of completion of the search | Examiner |
|---|---|---|
| THE HAGUE | 08-09-1989 | KASPERS H.M.C. |